# EUROPEAN PATENT SPECIFICATION

(11) **EP 2 615 928 B1**
(45) Date of publication and mention of the grant of the patent: **13.01.2016**
(21) Application number: 11771282.8
(22) Date of filing: 14.09.2011
(51) Int. Cl.: A23L 1/03, A23L 1/105, A23L 1/30, A23L 2/00, C12R 1/225, C12R 1/25, C12R 1/46, A23L 2/38, A23L 2/52, C12R 1/01

(54) **METHOD OF PRODUCTION OF FERMENTED, PRO-HEALTHY, CEREAL BEVERAGES**
VERFAHREN ZUR HERSTELLUNG VON FERMENTIERTEN GESUNDHEITSFÖRDERNDEN GETREIDEGETRÄNKEN
PROCÉDÉ POUR PRODUIRE DES BOISSONS FERMENTÉES AUX CÉRÉALES BONNES POUR LA SANTÉ

(30) Priority: 16.09.2010 PL 39242610
(43) Date of publication of application: 24.07.2013
(73) Proprietor: Instytut Biotechnologii Przemyslu Rolno-Spozywczego, 02-532 Warszawa (PL)
(72) Inventor: OWCZAREK, Lubomila, PL-03-982 Warszawa (PL); JASINSKA, Urszula T., PL-02-206 Warszawa (PL); SKAPSKA, Sylwia, PL-02-638 Warszawa (PL)
(74) Representative: Witek, Rafal
(86) International application number: PCT/PL2011/050036
(87) International publication number: WO 2012/036576

(56) References cited:
- EP-A1- 1 064 854
- PL-B1- 192 306
- US-A- 5 322 836
- US-A- 5 416 020
- SKAPSKA S ET AL: "Zmiany pojemnosci przeciwutleniajacej grzybow jadalnych w procesie kiszenia", ZMIANY POJEMNOSCI PRZECIWUTLENIAJACEJ GRZYBOW JADALNYCH W PROCESIE KISZENIA,, vol. 4, no. 59, 2008, pages 243-250, XP002664097,
- DATABASE BIOSIS [Online] BIOSCIENCES INFORMATION SERVICE, PHILADELPHIA, PA, US; 1995, WROBLEWSKA BARBARA ET AL: "The effect of selected microorganisms on the presence of immunoreactive fractions in cow and goat milks", XP002664330, Database accession no. PREV199598514793 & POLISH JOURNAL OF FOOD AND NUTRITION SCIENCES, vol. 4, no. 3, 1995, pages 21-29, ISSN: 1230-0322

## Description

The present invention relates to a method for production of fermented, pro-healthy, cereal beverages as replacers of fermented milks - especially for persons following the appropriate elimination diets.

The process of lactic fermentation is most commonly applied worldwide for production of beverages based on milk and other dairy products. However, these products are not suitable in nutrition of persons sensitized to milk protein and/or lactose intolerant. Due to beneficial influence on the human organism of Lactic Acid Bacteria (LAB), there is a constant growth of interest in new products obtained on the way of lactic fermentation, and especially as regards milk-less products that feature pro-healthy and even therapeutic values, provided that their sensory properties are positively perceived by the consumers.

In the paper on fermentation of pre-fermented and initially extruded rice flour" [Lee C.H. et al., Fermentation of pre-fermented and extruded rice flour by the lactic acid bacteria from sikhae. Food Biotechnology 1992, (6)3, 239-255] a method of producing fermented beverage from rice is described, that consists in 24-hour incubation at temperature 30°C of water suspension of the above mentioned rice preparation, inoculated with bacterial strains belonging to the species of *Lactobacillus plantarum* and *Leuconostoc mesenteroides*, that were isolated from sikhae (traditional Korean fish product). The purpose of the method application was obtaining a beverage of acceptable sensory features, what under the conditions of developed technology was not reached.

Patent GB2225922 describes the method of obtaining fermented cereal product Uji of repeatable and locally recognized sensory features, consisting at the first step in souring water suspension (ca. 45% d. m.) of ground corn or mixed cereal (corn/millet) seeds after inoculation with mesophyllic culture belonging to the *Lactobacillus* genus cultivated in nutritive medium; in replacement of the traditionally applied spontaneous fermentation. Product of 1-day fermentation at temperature 25°C is at the next step 6.7-fold diluted, up to 6.7% d. m., subjected to thermal processing at 100°C for 3 minutes in order to obtain the final product of gelatinized form and other expected features of Uji sensory profile. As a result, during the final thermal processing of soured cereal suspension, the final product is deprived of lactic acid bacteria.

EP 1064854 A1 discloses preparation of beverage made by fermenting a composition comprising sugar (3%), sodium citrate (0.05%) and cereal flour (4%) with 0.01 % *L.plantarum* until pH 3.5. The use of *S.thermophilus* and *L.bulgaricus* and mixtures thereof are also disclosed. The document relates to the process for increasing the fruity taste of foodstuffs with reduced content of fruit material, which comprises adding thereto an amylaceous composition resulting from a process wherein a slurry of cereal is first subjected to soaking and further sterilized and acidified and/or fermented.

Skapska S. et al. Zmiany pojemności przeciwutleniajacej grzybow jadalnych w procesie kiszenia, vol. 4, no. 59, 2008, pages 243-250, XP002664097 discloses the study of changes of antioxidant capacity and polyphenol content occurring during the processing of cultivated mushrooms using *L.planatarum* KKP 384 strain.

Wroblewska B. et al. "The effect of selected microorganisms on the presence of immunoreactive fractions in cow and goat milks", Polish Journal of Food and Nutrition Sciences, vol. 4, no. 3, 1995, pages 21-29, describes a study of the effect of selected cocci, including *Streptococcus salivarius* ssp. *thermopihilus* TkM3, on animal substrate (the presence of immunoreactive fractions in milk).

Polish patent application no. P. 349 543 A1 relates to the strain *L.bulgaricus* KKP 2007/p as producing exclusively L(+) isomer of lactic acid.

US 5322836 A discloses *Bifidobacterium breve* ATTC 15700 and *Bifidobacterium infantis* ATTC 15697 and is directed to the growth-promoting agents as, e.g. bovine lactoferrin and compositions comprising them. Bifidobacteria as probiotic microorganisms, are introduced to soured products in order to supplement biota of fermented beverages.

From the publication on administration of different strains of *Lactobacillus* genus in fermented oatmeal soup, and moreover colonization *in vivo* of human intestinal mucosa as well as the colonization effect on indigenous micro-flora [Johansson M.-L. et al., Administration of different Lactobacillus strains in fermented oatmeal soup: in vivo colonization of human intestinal mucosa and effect on the indigenous flora. Applied and Environmental Microbiology 1993, 59, 1, 15-20] the therapeutic beverage is known, obtained on way of souring of oat preparations with bacterial monocultures belonging to the *Lactobacillus* genus, that were isolated from human intestinal mucosa (e.g. *L. plantarum* 299), rat intestinal mucosa (e.g. *L. reuteri* 47) or sour dough (e.g. *L. plantarum* 299v), freeze-drying of 19 monoculture fermentation products and their combination resulting in the obtainment of final product in the form of dry preparation of content 7.4 log cfu·g⁻¹ of mixed cultures of *Lactobacillus* genus. The goal of obtaining this product, was testing the option to colonize human intestinal mucosa by live bacterial cultures after oral administration of the reconstituted aforementioned beverage to volunteers. The expected option was confirmed statistically only with reference to the *L. plantarum* 299v culture.

Within the last decade, progress of knowledge took place concerning the benefits of lactic fermentation in many additional aspects except for always valued preservation advantages of the process and its valuable sensory effects.

The major product of lactic fermentation and important constituent of fermented cereal beverages is lactic acid. In nutrition, especially of children, the most important thing is the form of lactic acid given in the diet. Since 1974 FAO/WHO [*Food and Agricultural Organization* /*World Health Organization*] recommends the adults to limit the consumption of D(-) optical isomer of lactic acid, one of two naturally occurring stereo isomers of lactic acid, to 100 mg per day. In case of fermented products for children nutrition, however, the only acceptable form of lactic acid is L(+) isomer. Presence of D(-) lactate in fermented products for newborns and children is perceived as highly unwanted. This can be explained by poor potential of utilization of D(-) lactic acid isomer in child's organism and the risk of occurring of D(-) acidose, particularly in populations of children with short bowel syndrome, described in the paper "Probiotic therapy in children with short bowel syndrome and bacterial overgrowth" [Young R., Vanderhoof J.A., Probiotic therapy in children with short bowel syndrome and bacterial overgrowth. Poster at the Annual Meeting of American Gastroenterology Association. 11-14 May 1997, Washington DC, USA].

Most of the microorganisms species applied in the industry for fermentation of food, including many probiotic cultures of *Lactobacillus,* produce D(-) as well as L(+) isomers of lactic acid what is reflected in the composition of the final products of fermentation.

They are known from publications "Lactic Acid Bacteria. Classification and Physiology" [Axelsson L., Lactic Acid Bacteria: Classification and Physiology. In: Lactic Acid Bacteria. Microbial and Functional Aspects. Eds.: Salminen S., Wright von A., Ouvehand A., A series of Food Science and Technology. Marcel Dekker, Inc., New York, Basel 2004, pp. 1-66] distinct, dependent on the lactate dehydrogenase (D-nLDH or L-nLDH) availability in the microflora of lactic fermentation, species' predispositions of microorganisms used in the production of fermented food, for development of two naturally occurring stereo isomers in variants: exclusively L(+) isomer of lactic acid, exclusively D(-) isomer of lactic acid, approximately equal quantities of both isomers or mix of both with predominating content of one of them, but at measurable content of the other. In the cases of fermentations with participation of some of the lactic acid bacteria, a dependence of induction of lactate racemase formation on the presence of the created L(+) lactic acid is known, as a result of which there is a progressing conversion of form L(+) to D(-) of lactic acid, and in effect the occurrence of mix of D(-) and L(+) isomers of lactic acid in the fermentation products [Sakai K., Fujii N., Chukeatirote E., Racemization of L-lactic acid in pH-swing open fermentation of kitchen refuse by selective proliferation of Lactobacillus plantarum. Journal of Bioscience and Bioengineering 2006, 102, 3, 227-232].

However, the achieved, as a result of fermentation, final output of both isomeric forms of lactic acid, their optical purity and occurring changes in the composition proportions may be influenced also by the following factors: medium composition [Iino T. et al.; The effect of sodium acetate on the activity of L- and D-lactate dehydrogenases in Lactobacillus sakei NRIC 1071T and other lactic acid bacteria. J. Gen. Appl. Microbiology 2003 49(1): 51-58, as well as: Hofvendahl K.&Hahn-Hagerdal B.; L-Lactic acid production from whole wheat flour hydrolysate using strains of Lactobacilli and Lactococci. Enzyme and Microbial Technology 1997, 20, 4, 301-307], physical conditions of incubation [Qing-Jin et al.; Effect of lactic acid bacteria on D(-) and L(+) lactic acid contents of kimchi. Food Science and Biotechnology 2006, 15, 6, 948-953] as well as the method of handling the fermentation products, e.g. introduction of additives to soured products [Lutomski L., Importance of lactic acid and its derivatives in diet and therapy. In: Application of lactic acid and its derivatives. Edited by: M. Jankiewicz, PTTZ (Polskie Towarzystwo Technologow Zywnosci - Polish Association of Food Technologists) - Great Poland Branch, 1995, 59-71].

Except for some relatively ananerobic strains of lactic acid producing bacteria belonging to the *Lactobacillus* and *Streptococcus* genera, also the anaerobic bifidobacteria are lactic acid producers, that represent physiological microorganisms of intestinal biota reacting competitively in the gastrointestinal tract with pathogenic bacteria. They are called probiotics. Stimulators of these probiotic microorganisms are prebiotic substances, oligosaccharides of raw materials of plant origin, e.g. these present in cereals, seeds of leguminous plants etc. Combinations of probiotics with prebiotics in foodstuffs, manifesting synergistic action in organism, are called synbiotics [Socha J., Stolarczyk A., Socha P., Administration of bifidobacteria in prophylaxis and treatment of selected children diseases. Contemporary Pediatry, Gastroenterology, Hepatology and Children Nutrition, 2002, 4, 1, 43-47].

The purpose of the invention is development of the production method of fermented, pro-healthy cereal beverages without constituents of milk, that represent the source of beneficial LABs or LABs and bifidobacteria, with the possibly highest share of L(+) isomer in the total lactic acid content, that enable satisfactory sensory and nutritionally safe application in place of fermented milk in human nutrition, especially of persons with allergies to milk protein or lactose intolerant. These fermented cereal beverages containing exclusively L(+) isomer of lactic acid, are fit to be used in child nutrition.

The object of the present invention is the method for production of fermented, pro-healthy cereal beverages consisting in preparing of cereal preparation for fermentation, its thermal preservation, inoculation with the strains of lactic acid bacteria (LAB) and fermentation, characterized in that in aqueous solution of 2-8% by weight of disaccharide not lactose, 0-6% by weight of monosaccharide and 0.05-0.25% by weight of sodium citrate - a suspension 3-8% by weight is created of the cereal processed product, preferably flour, that is inoculated with the lactic acid bacteria strains selected from *Lactobacillus plantarum* KKP 2025p strain or two-strains set of *Lactobacillus plantarum* KKP 2025p and *Streptococcus thermophilus* KKP 2030p cultures or with the *Lactobacillus bulgaricus* s/l KKP 2007/p strain in quantity 0.001-0.01% by weight in case of each concentrated, lyophilized culture, conducing the fermentation to pH not higher than 4.6, wherein the obtained product maintains at least 7 log cfu g⁻¹ of bacterial cells including bifidobacteria within 28 days of storage at temperature 4 °C.

Preferably, the method of invention is characterized in that into the fermentation product a biotum supplement is introduced in the form of concentrated lyophilized culture of *Bifidobacterium breve* KKP 2028p or *Bifidobacterium infantis* KKP 2029p, in quantity 0.01-0.15% by weight.

According to the invention, as a cereal raw material, preferably rice and/or oat or buckwheat is used.

In the method according to the invention, the fermentation is performed preferably at a temperature of 37-43°C.

The another object of the invention is use of the strains selected from the group consisting of:
- *Lactobacillus plantarum* deposited in the Collection of Industrial Microorganisms IAFB under the accession number KKP 2025p,
- two-strains set of *Lactobacillus plantarum* deposited in the Collection of Industrial Microorganisms IAFB under the accession number KKP 2025p and *Streptococcus thermophilus* TKM3 deposited in the Collection of Industrial Microorganisms IAFB under the accession number KKP 2030p,
- *Lactobacillus bulgaricus* s/l deposited in the Collection of Industrial Microorganisms IAFB under the accession number KKP 2007/p,
for production of a fermented, pro-healthy cereal beverage.

Bacterial strains used in the invention: *Lactobacillus bulgaricus* s/l KKP/2007/p and *Lactobacillus plantarum* KKP 384 are deposited at the International Collection of Industrial Microorganisms of the Institute of Agricultural and Food Biotechnology (IAFB). *Streptococcus thermophilus* T_{K}M₃ strain is derived from the collection of the Microbiology Department of the Institute of Animal Reproduction and Food Research, PAN (Polish Academy of Sciences), Olsztyn. *Bifidobacterium breve* ATCC 15700 and *Bifidobacterium infantis* ATCC 15697 strains are cultures of American Type Culture Collection.

Said strains are presented in Table I.

**Table I**

| No. | Deposit no. assigned by IAFB | Strain |
|---|---|---|
| 1 | KKP 2025p | *Lactobacillus plantarum* KKP384 |
| 2 | KKP 2030p | *Streptococcus thermophilus* T_{K}M₃ |
| 3 | KKP 2007/p | *Lactobacillus bulgaricus* s/l KKP 2007/p |
| 4 | KKP 2028p | *Bifidobacterium breve* ATTC 15700 |
| 5 | KKP 2029p | *Bifidobacterium infantis* ATTC 15697 |

Unexpectedly, it was found out that the applied two-strains set of starter cultures (*Lactobacillus plantarum* KKP 384 and *Streptococcus thermophilus* T_{K}M₃) used in the invention, thanks to synergistic action, contributes to the 50% shortening of time needed to sour the preparation. As it occurred during souring the preparation with 2-strain starter set, the adopted fermentation temperature closed to the optimum for one of the starter cultures, not only accelerates the fermentation process, but also does not limit the development and growth of the other strain in the product being soured.

Unexpectedly, it was found favorable, that oat and oat-rice preparations prepared in accordance with the method according to the invention, enable the starter monoculture *Lactobacillus bulgaricus* s/l KKP/2007/p to produce L(+) isomer of lactic acid exclusively during fermentation process.

Unexpectedly, it was found favorable, that application in the invention of *Bifidobacterium* culture as the supplement of biotum of the beverage fermented with participation of *Lactobacillus bulgaricus* s/l KKP/2007/p does not influence the transformation of the created L(+) isomer of lactic acid in the beverage.

It was also found favorable, that environment of oat and oat-rice beverage soured with participation of *Lactobacillus bulgaricus* s/l KKP/2007/p, after introduction into the beverage biota of the supplement in the form of bifidobacteria, *Bifidobacterium infantis* ATCC 15697 or *Bifidobacterium breve* ATCC 15700, enables culture cohabitation and does not influence the transformation of L(+) isomer of lactic acid developed in the beverages, providing maintenance of 94-96% share of this isomer in the total content of lactic acid of beverage until 28^{th} day of cold storage of products at temperature 4°C.

In the method according to the invention, a formula was developed of simple cereal preparations enabling one to receive aqueous suspensions of cereal products representing different nutritional and pro-healthy values, composing various sources of prebiotic soluble fiber, oligosaccharides, vitamins of group B, being free of gluten (rice, buckwheat) or containing traces of similarly acting avenin (oat). The developed cereal preparations performed as flour suspensions in the saccharide solutions, alike the products of their fermentation that should substitute fermented milk, are close in texture to milk yoghurts and composition of the preparations provide buffer capacity that enable souring by lactic acid bacteria. The described effect is achieved at economically justified concentration of starter cultures in the inoculum that provide rational duration of the fermentation process, achievement and maintenance in the final products, within 28 days of storage at temperature 4°C, of high numbers' populations of bacterial cells including bifidobacteria (no less than 7 log cfu g⁻¹) and acceptable sensory profile.

The invention is closer illustrated by embodiments not limiting its scope.

### Example I

The method of producing of fermented rice beverage containing bifidobacteria and the mix of L(+) and D(-) isomers of lactic acid.

Preparation for fermentation was performed as the aqueous suspension of 5% by weight of rice flour in aqueous solution of mix of 5% by weight of saccharose and 0.2% by weight of sodium citrate dihydrate. Starch in the aqueous solution of the preparation was pasted/gelatinized at temperature 80°C within 7 min. The rice preparation, after pasting, was sterilized for 15 min. at temperature 121°C, cooled down to 37°C and then two synergistically cooperating, concentrated, lyophilized cultures of *Lactobacillus plantarum* KKP384 and *Streptococcus thermophilus* T_{K}M₃ were introduced, each in quantity 0.01% by weight.

The rice preparation mixed with the starter cultures was fermented at temperature 37°C for about 20 hours until achieving pH not higher than 4.6.

Culture of *Bifidobacterium breve* ATCC 15700, in quantity 0.10% by weight of concentrated lyophilized form, was added to the soured rice preparation, as a supplement of starter micro-flora contained in it, in order to obtain the final product that was poured to glass unitary packages and placed in the cold store at temperature 4°C.

During storage under cold conditions, the prohealthy rice beverage was characterized by the change of parameters related to the acidity: drop of pH value, increase of titratable acidity and the total content of lactic acid, including L(+) and D(-) isomers of lactic acid. The fermented rice beverage, under cold storage conditions, was characterized by slight note of flavor typical for pasted rice with delicately increasing notes of sour odour and taste, and moreover, smooth, uniform, relatively dense, not subject to changes, texture of fluid cereal paste. The sensory profile of the product, perceived as harmonized within the scope of sweet and sour notes of taste, was well evaluated within the attributes of flavor (odour and taste) and texture until the end of the 28-day's storage period at temperature 4°C.

Content of lactic acid bacteria belonging to the *Lactobacillus* and *Streptococcus* genera amounted in the fresh product 8.3 and 8.1 log cfu·g⁻¹ and in the product after 28-day's storage 7.6 and 7.3 log cfu g⁻¹, respectively.

Population number of bacterial culture belonging to the *Bifidobacterium* genus in the beverage changed during the 28-day's storage within the range 8.0-6.0 log cfu·g⁻¹.

Content of lactic acid amounting in fresh product 0.130 g/100g, after 28 days of cold storage reached 0.190 g/100g, and the share of L(+) stereo isomer in the total content of lactic acid in the beverage changed during the 28-days' cold storage of beverage, dropping within the range 66-55%.

### Example II

The method of producing of fermented buckwheat beverage containing the mix of L(+) and D(-) isomers of lactic acid.

Preparation for fermentation was performed as the aqueous suspension of 5.5% by weight of buckwheat flour in aqueous solution of mix of 3% by weight of saccharose and 0.2% by weight of sodium citrate dihydrate. Starch in the aqueous solution of the preparation was pasted/gelatinized at temperature 80°C within 7 min. The buckwheat preparation after pasting was sterilized for 15 min at temperature 121°C, cooled down to 43°C and concentrated lyophilized culture of *Lactobacillus plantarum* KKP384 was introduced in quantity 0.004% by weight.

The preparation mixed with the starter culture was fermented at temperature 43°C for about 12 hours until souring to pH not higher than 4.6.

The final product was poured into glass unitary packages and placed in cold store at temperature 4°C.

During storage under cold conditions, the prohealthy buckwheat beverage was characterized by the change of parameters related to the acidity: drop of pH value, increase of titratable acidity and the total content of lactic acid, including L(+) and D(-)isomers of lactic acid.

The fermented buckwheat beverage, during storage, was characterized by flavor typical for buckwheat groats with increase of sour notes of odour and taste. General sensory profile of the product, with harmony of sweet and sour notes of taste, runny texture of cereal paste was perceived and well evaluated until the end of the 28-day storage period.

Content of lactic acid bacteria belonging to the *Lactobacillus* genus was 8.8 log cfu·g⁻¹ in the fresh product and did not drop below 8.3 log cfu·g⁻¹ during 28-days' cold storage.

Content of lactic acid about 0.225 g/100g in the fresh product, after 28 days of cold storage reached the value of 0.460 g/100g, however the share of L(+) isomer of lactic acid in the total lactic acid content did not significantly change but stabilized at the level close to 60%.

### Example III

The method of producing of fermented oat beverage containing bifidobacteria and L(+) isomer of lactic acid exclusively.

Preparation for fermentation was performed as the aqueous suspension of 4% by weight of oat flour in aqueous solution of mix of 4% by weight of glucose, 2.5% by weight of saccharose and 0.2% by weight of sodium citrate dihydrate. Starch in the aqueous solution of the preparation was pasted/gelatinized at temperature 80°C within 7 min. The oat preparation after pasting was sterilized for 15 min at temperature 121°C, cooled down to 37°C and concentrated lyophilized culture of *Lactobacillus bulgaricus* s/l KKP/2007/p was introduced in quantity 0.005% by weight.

The preparation, after inoculation with the starter culture, was fermented at temperature 37°C for 10 hours until souring to pH 4.6.

The culture of *Bifidobacterium infantis* ATCC 15697 was added to the fermentation product as a supplement of the starter culture, in concentrated lyophilized form, in quantity 0.03% by weight. The obtained final product was poured into glass unitary packages and placed in cold store at temperature 4°C.

During storage under cold conditions, the pro-healthy oat beverage was characterized by the change of parameters related to the acidity: drop of pH value, increase of titratable acidity and the total content of lactic acid, here present in the form of L(+) lactic acid isomer exclusively.

The fermented oat beverage, during the storage, was characterized by slight walnut note of flavor, typical for oat paste, with delicately increasing while in storage, predominating notes of sour odour and taste. General sensory profile of the product, with harmony of sweet and sour notes of taste, relatively dense, smooth texture of runny cereal paste with glossy surface, was perceived and well evaluated until the end of the 28-days' storage period.

Content of lactic acid bacteria belonging to the *Lactobacillus* genus was 8.2 log cfu·g⁻¹ in the fresh product and did not drop below 7.4 log cfu·g⁻¹ during 28-days' cold storage. Population number of bacteria belonging to the *Bifidobacterium* genus did not drop in the beverage during the cold storage period below 6.0 log cfu·g⁻¹.

Content of lactic acid about 0.195g/100g in the fresh product, after 28 days of storage reached the value of 0.485g/100g, however the share of L(+) isomer of lactic acid in the total lactic acid content did not change and stabilized at the level close to 96% by weight during the full storage period.

### Example IV

The method of producing of oat-rice beverage containing bifidobacteria and L(+) isomer of lactic acid exclusively.

Preparation for fermentation was prepared as the aqueous suspension of 5% by weight of oat and rice flours in proportion 1:1 in aqueous solution of mix of 4% by weight of glucose, 2.5% by weight of saccharose and 0.2% by weight of sodium citrate dihydrate. After pasting the starch of the preparation at temperature 80°C, preparation sterilization at temperature 121°C and its cooling down to 37°C, concentrated lyophilized culture of *Lactobacillus bulgaricus* s/l KKP/2007/p was added in 0.01% quantity by weight.

The preparation mixed with the starter culture, was fermented at temperature 37°C for 11 hours until souring to pH 4.6.

Culture of *Bifidobacterium breve* ATCC 15700 was added to the fermentation product as a supplement of the starter culture, in concentrated lyophilized form, in quantity 0.10% by weight.

The obtained beverage was poured into glass unitary packages and placed in cold store at temperature 4°C.

During storage under cold conditions, the pro-healthy oat-rice beverage was characterized by the change of parameters related to the acidity: drop of pH value, increase of titratable acidity and the total content of lactic acid, here present in the form of L(+) isomer of lactic acid exclusively.

The fermented oat-rice beverage, during the storage, was characterized by flavor typical for soured cereal paste, with delicately increasing while in storage, predominating notes of sour odour and taste. The general sensory profile of the product, characterized by a harmony of sweet and sour notes of taste, relatively dense, uniform texture of liquid cereal paste, was well evaluated until the end of 28-days' storage within the scope of attributes of flavor (odour and taste) and texture.

Content of lactic acid bacteria belonging to the *Lactobacillus* genus and bifidobacteria cells in the beverages was 8.0 log cfu·g⁻¹ in the fresh product and did not drop below 7.0 and 6.0 log cfu·g⁻¹, respectively, during 28-day's cold storage.

Content of lactic acid amounting about 0.190 g/100g in the fresh product, after 28 days of storage reached the value of 0.470 g/100g, however the share of L(+)stereo isomer of lactic acid in the total lactic acid content maintained at the level close to 96% during the full storage period.

## Claims

1. A method for production of fermented, pro-healthy cereal beverages consisting in preparing of the cereal preparation for fermentation, its thermal preservation, inoculation with the strains of lactic acid bacteria (LAB) and fermentation, **characterized in that** in aqueous solution of 2-8% by weight of disaccharide not lactose, 0-6% by weight of monosaccharide and 0.05-0.25% by weight of sodium citrate - a suspension 3-8% by weight is created of the cereal processed product, preferably flour, that is inoculated with the lactic acid bacteria strains selected from *Lactobacillus plantarum* KKP2025p strain or two-strains set of *Lactobacillus plantarum* KKP2025p and *Streptococcus thermophilus* KKP2030p cultures or with the *Lactobacillus bulgaricus* s/l KKP 2007/p strain, in quantity 0.001-0.01% by weight in case of each concentrated, lyophilized culture, conducing the fermentation to pH not higher than 4.6, wherein the obtained product maintains at least 7 log cfu g⁻¹ of bacterial cells including bifidobacteria within 28 days of storage at temperature 4°C.

2. The method according to claim 1, **characterized in that** into the fermentation product a biotum supplement is introduced in the form of concentrated lyophilized culture of *Bifidobacterium breve* KKP 2028p or *Bifidobacterium infantis* KKP 2029p, in quantity 0.01-0.15% by weight.

3. The method according to claim 1, **characterized in that** rice and/or oat or buckwheat is used as a cereal raw material.

4. The method according to the claim 1, **characterized in that** the fermentation is performed at temperature 37- 43°C.

5. Use of the strains selected from the group consisting of:
a) *Lactobacillus plantarum* deposited in the Collection of Industrial Microorganisms IAFB under the accession number KKP 2025p,
b) two-strains set of *Lactobacillus plantarum* deposited in the Collection of Industrial Microorganisms IAFB under the accession number KKP 2025p and *Streptococcus thermophilus* TKM3 deposited in the Collection of Industrial Microorganisms IAFB under the accession number KKP 2030p,
c) *Lactobacillus bulgaricus* s/l deposited in the Collection of Industrial Microorganisms IAFB under the accession number KKP 2007/p,
for production of a fermented, pro-healthy cereal beverage.

## Patentansprüche

1. Verfahren zur Herstellung von fermentierten gesundheitsfördernden Zerealiengetränken, bestehend aus der Präparation der Zerealienpräparation zur Fermentation, seiner thermischen Konservierung, Beimpfung mit den Stämmen von Milchsäurebakterien (MSB) und Fermentation, **dadurch gekennzeichnet, dass**
in einer wässrigen Lösung von 2 - 8 Gew% an Disaccharid, nicht Lactose, 0 - 6 Gew% an Monosaccharid und 0,05 - 0,25 Gew% an Natriumzitrat - eine Suspension von 3 - 8 Gew% des zu verarbeitenden Zerealienprodukts, bevorzugt Mehl, erzeugt wird, die is inoculated mit den Milchsäurebakterien Stämmen ausgewählt aus *Lactobacillus plantarum* KKP 2025p Stamm oder einem zwei Stämme-Set von *Lactobacillus plantarum* KKP 2025p und *Streptococcus thermophilus* KKP 2030p Kulturen oder mit dem *Lactobacillus bulgaricus* s/l KKP 2007/p Stamm beimpft wird, in einer Menge von 0,001 - 0,01 Gew% im Falle jeder konzentrierten lyophilisierten Kultur, einstellen der Fermentation auf einen pH von nicht mehr als 4,6, wobei das erhaltene Produkt mindestens 7 log cfu g⁻¹ an bakteriellen Zellen einschließlich Bifidobakterien innerhalb von 28 Tagen bei einer Lagerung bei einer Temperatur von 4°C beibehält.

2. Verfahren nach Anspruch 1, **dadurch gekennzeichnet, dass** eine Biotum-Unterstützung in Form einer konzentrierten lyophilisierten Kultur von *Bifidobacterium breve* KKP 2028p oder *Bifidobacterium infantis* KKP 2029p in einer Menge von 0,01 - 0,15 Gew% in das Fermentationsprodukt eingeführt wird.

3. Verfahren nach Anspruch 1, **dadurch gekennzeichnet, dass** Reis und/oder Hafer oder Buchweizen als ein Zerealienrohmaterial verwendet wird.

4. Verfahren nach Anspruch 1, **dadurch gekennzeichnet, dass** die Fermentation bei einer Temperatur von 37 - 43°C durchgeführt wird.

5. Verwendung der Stämme ausgewählt aus der Gruppe bestehend aus:
a) *Lactobacillus plantarum,* wie hinterlegt in der Sammlung von Industriellen Mikroorganismen IAFB unter der Zugangsnummer KKP 2025p,
b) einem zwei Stämme-Set von *Lactobacillus plantarum,* wie hinterlegt in der Sammlung von Industriellen Mikroorganismen IAFB unter der Zugangsnummer KKP 2025p und *Streptococcus thermophilus* TKM3, wie hinterlegt in der Sammlung von Industriellen Mikroorganismen IAFB unter der Zugangsnummer KKP 2030p,
c) *Lactobacillus bulgaricus* s/l, wie hinterlegt in der Sammlung von Industriellen Mikroorganismen IAFB unter der Zugangsnummer KKP 2007/p,
zur Herstellung eines fermentierten gesundheitsfördernden Zerealiengetränks.

## Revendications

1. Procédé pour la production de boissons aux céréales fermentées bonnes pour la santé consistant à préparer la préparation de céréales pour la fermentation, sa conservation thermique, l'inoculation des souches de bactéries lactiques (BL) et la fermentation, **caractérisé en ce que** dans une solution aqueuse de 2-8 % en poids de disaccharide non lactose, de 0-6 % en poids de monosaccharide et de 0,05-0,25 % en poids de citrate de sodium - une suspension de 3-8 % en poids est créée du produit céréalier traité, de préférence la farine, à laquelle on inocule les souches de bactéries lactiques sélectionnées parmi la souche de *Lactobacillus plantarum* KKP2025p ou un ensemble de deux souches de cultures de *Lactobacillus plantarum* KKP2025p et de *Streptococcus thermophilus* KKP2030p ou avec la souche de *Lactobacillus bulgaricus* s/l KKP 2007/p, en une quantité de 0,001-0,01 % en poids dans le cas de chaque culture concentrée lyophilisée, ce qui conduit à la fermentation à un pH qui ne dépasse pas 4,6, dans lequel le produit obtenu conserve au moins 7 log ufc g⁻¹ de cellules bactériennes comprenant des bifidobactéries au cours de 28 jours qui suivent l'entreposage à une température de 4°C.

2. Procédé selon la revendication 1, **caractérisé en ce que** dans le produit de fermentation un supplément de biotum est introduit sous la forme d'une culture lyophilisée concentrée de *Bifidobacterium breve* KKP 2028P ou de *Bifidobacterium infantis* KKP 2029p, en une quantité de 0,01-0,15 % en poids.

3. Procédé selon la revendication 1, **caractérisé en ce que** du riz et/ou de l'avoine ou du blé noir sont utilisés en tant que matière première céréalière.

4. Procédé selon la revendication 1, **caractérisé en ce que** la fermentation est effectuée à une température de 37-43°C.

5. Utilisation des souches sélectionnées dans le groupe constitué par :
a) *Lactobacillus plantarum* déposée dans la collection de micro-organismes industriels IAFB sous - le numéro d'accession KKP 2025p,
b) ensemble de deux souches de *Lactobacillus plantarum* déposée dans la collection de micro-organismes industriels IAFB sous le numéro d'accession KKP 2025p et *Streptococcus thermophilus* TKM3 déposée dans la collection de micro-organismes industriels IAFB sous le numéro d'accession KKP 2030/p,
c) *Lactobacillus bulgaricus* s/l déposée dans la collection de micro-organismes industriels IAFB sous le numéro d'accession KKP 2007/p,
pour la production d'une boisson aux céréales fermentée bonne pour la santé.
